# EUROPEAN PATENT APPLICATION

(11) **EP 1 886 571 A2**
(43) Date of publication of application: **13.02.2008**
(21) Application number: 07112824.3
(22) Date of filing: 20.07.2007
(51) Int. Cl.: A21D 8/04, C12N 1/18

(54) **Method for producing natural yeast for bakery products and yeast obtained with the method**

(30) Priority: 31.07.2006 IT PD20060298
(71) Applicant: Dal Colle, Egidio, 37030 San Pietro di Lavagno (IT)
(72) Inventor: Dal Colle, Egidio, 37030 San Pietro di Lavagno (IT)
(74) Representative: Modiano, Micaela Nadia

(57) **Abstract**

A method for producing natural yeast for bakery products, comprising the steps of:
a) crumbling a natural yeast (mother yeast) with the addition of flour by using a mixing machine;
b) maturation of the mixture for a period of 4-8 hours at room temperature;
c) granulation;
d) cooling at a temperature between -20 and -35 °C;
e) packaging and storage at a low temperature between -18 and -20 °C.

The resulting yeast has the appearance of a granulated substance (conveniently, the size is the same as rice), which is distributed in frozen form and is thawed at the time of use in the chosen amount.

## Description

The present invention relates to a method for producing a natural yeast for bakery products and to a yeast obtained with the method.

Natural yeast is a compound obtained from the fermentation of a mixture of flour and water.

This compound contains microorganisms of various species of the Saccharomyces, Lactobacillus and Streptococcus genera.

These microorganisms feed on simple and complex sugars such as the ones contained in flour starch.

In biological activity, this is known as fermentation and is used in the process for producing leavened doughs.

The microorganisms convert sugars in particular into gaseous carbon dioxide, which generates an increase in the volume of the mix, in which small cavities form which enclose said carbon dioxide.

The porous mix obtained during baking generates a soft, pliable product.

Moreover, yeast microorganisms, during fermentation, produce substances which characterize the flavor and aroma of the product and also increase its preservation time.

It is evident that all this is affected by the quantity of live microorganisms contained in the yeast.

However, it is also known that during the preparation of the yeast and during its storage, a large percentage of these microorganisms dies, and therefore the yield of the yeast decreases, and this requires the use of a larger quantity of yeast with an increase in costs.

The aim of the present invention is to provide a method for producing a natural yeast which contains a large quantity of live microorganisms.

A second consequent object is to obtain a yeast which is very active in the fermentation process.

Another object is to obtain a yeast which generates, during the fermentation of the product in which it is used, a significant quantity of substances which induce flavor and aroma.

Another object is to provide a yeast which can be stored for long periods before use without losing its characteristics.

Another object is to provide a method which can be performed with machines which are already known in the field.

This aim and these and other objects, which will become better apparent hereinafter, are achieved by a method for producing natural yeast for bakery products, characterized in that it comprises the steps of:
a) crumbling a natural yeast (mother yeast) with the addition of flour by using a mixing machine;
b) maturation of the mixture for a period of 4-8 hours at room temperature;
c) granulation;
d) cooling at a temperature between -20 and -35 °C;
e) packaging and storage at a low temperature between -18 and -20 °C.

The new method which seeks to obtain a yeast which is improved in its characteristics and is adapted for use for bakery products comprises a succession of operating steps, the essential ones being given in the description that follows.

The first step provides for the crumbling of a natural yeast (mother yeast) with the addition of flour by using a mixing machine for example of the spiral type.

It is of course possible to use other mixing machines which perform equivalent processes.

After mixing has occurred, the product is allowed to rest in order to achieve maturation, indicatively for 4-8 hours at a room temperature which can be indicatively between 20 and 25 °C.

Of course, both the indicated times and the indicated temperatures are the ones which optimize industrial production, but different times and different temperatures, which are in any case mutually correlated, do not alter the process, and maturation is controlled by means of repeated analyses.

Once maturation has occurred, granulation can be performed in a cool environment with a granulating-calibrating machine, for instance at a temperature below the room temperature, for instance at a temperature less than 30°C, preferably less than 25° or less than 20°C.

These granulating-calibrating machines are known in the food sector and in this case granulation occurs until a granule size comparable to a rice grain is obtained.

In this case also, the size of the grain is not critical, but the indicated size is the one that is best suited for the use that the user will make of it during the preparation of bakery products.

Then, once granulation has been completed, the mixture is cooled to a temperature comprised between -20 and -35 °C, more preferably between -25°C and -35°C, by using machines which are known in the field as refrigeration machines or cryogenic gas tunnels.

This step ends when all of the product is uniformly brought to a temperature between -20 and -35 °C or, more preferably, between -25°C and -35°C.

In this step it is important not to go too far below -35 °C, since already at -40 °C the product undergoes a stress which damages it.

In a second embodiment of the invention, the granulation can be performed only after the cooling step. In this case, the frozen product can be granulated with a granulating-calibrating machine, always keeping it at the temperatures between -20°C and -35°C cited above.

In any case, regardless of whether granulation occurs before or after the cooling step, the product, still kept at low temperature, is packaged and distributed in frozen form at a temperature of approximately -18 to -20 °C.

At the time of use, the user takes the necessary amount of frozen yeast and uses it in the mixture that he/she must prepare, while the remaining part is always kept at freezing temperature, which allows to preserve for long times the yeast that has been obtained with the method, since the treatment and preservation at low temperature stop fermentation and keep the enzymes alive, and allows said enzymes, after thawing and during use, to be active in a much higher percentage than observed in normal yeasts.

This allows to obtain much softer and more fragrant bakery products.

Of course, as already mentioned, this description of the method and of the product and the means for performing the method is a non-limiting example and is suitable to allow the person skilled in the art, with his/her knowledge, to perform the method and obtain the product.

The disclosures in Italian Patent Application No. PD2006A000298 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A method for producing natural yeast for bakery products, **characterized in that** it comprises the steps of:
a) crumbling a natural yeast (mother yeast) with the addition of flour by using a mixing machine;
b) maturation of the mixture for a period of 4-8 hours at room temperature;
c) granulation;
d) cooling at a temperature between -20 and -35 °C;
e) packaging and storage at a low temperature between -18 and -20 °C.

2. The method according to claim 1, **characterized in that** the granulation of step c) is carried out before the cooling step d) and in a cool environment, preferably at a temperature less than the temperature of the maturation environment of step b).

3. The method according to claim 2, **characterized in that** said cooling is performed at a temperature between -25°C and -35°C.

4. The method according to claim 1, **characterized in that** the temperature of the maturation environment is comprised between 20 and 30 °C.

5. The method according to claim 1, **characterized in that** maturation is controlled by analysis.

6. The method according to claim 1, **characterized in that** the cooling of item d) is obtained with a refrigeration machine.

7. The method according to claim 1, **characterized in that** the cooling of item d) is obtained with a cryogenic gas tunnel.

8. The method according to claim 1, **characterized in that** the granulation of item c) brings the product to a granule size substantially equal to a rice grain.

9. The method according to claim 1, **characterized in that** said granulation is performed after said cooling step d) and at a temperature between -20 and -35 °C.

10. Natural yeast obtained from the method according to claim 1, **characterized in that** it is provided in frozen granules at a temperature between-18 and-20 °C.
